# EUROPÄISCHE PATENTANMELDUNG

(11) **EP 1 495 679 A1**
(43) Veröffentlichungstag der Anmeldung: **12.01.2005**
(21) Anmeldenummer: 04021804.2
(22) Anmeldetag: 05.04.2000
(51) Int. Cl.: A23B 4/12, A23B 4/22, A23K 3/00, A23K 3/03, C12R 1/46

(54) **Schutzkulturen und deren Verwendung bei der Konservierung von Lebensmitteln**

(30) Priorität: 09.04.1999 DE 19917715
(62) Teilanmeldung aus: 00918851.7
(71) Anmelder: DANISCO A/S, 1001 Copenhagen K. (DK)
(72) Erfinder: Elsser, Dieter, Dr., 25842 Bargum (DE)
(74) Vertreter: UEXKÜLL & STOLBERG

(57) **Zusammenfassung**

Die vorliegende Erfindung betrifft neuartige Milchsäurebakterien umfassende Schutzkulturen, welche zur Konservierung von Lebensoder Futtermitteln dienen können, die auch unter Kühlung nur begrenzt haltbar sind. Die Schutzkulturen sind in der Lage, bei Unterbrechung der Kühlkette oder bei Nichteinhaltung der vorgeschriebenen Kühltemperatur das Wachstum von für den Verbraucher gefährlichen Bakterien zu hemmen. Ferner werden Lebensoder Futtermittel zur Verfügung gestellt, welche die erfindungsgemäßen Schutzkulturen enthalten.

## Beschreibung

Die vorliegende Erfindung betrifft neuartige Milchsäurebakterien umfassende Schutzkulturen, welche zur Konservierung von Lebensoder Futtermitteln dienen können, die auch unter Kühlung nur begrenzt haltbar sind. Die Schutzkulturen sind in der Lage, bei Unterbrechung der Kühlkette oder bei Nichteinhaltung der vorgeschriebenen Kühltemperatur das Wachstum von für den Verbraucher gefährlichen Bakterien zu hemmen.

Bestimmte Lebens- oder Futtermittel wie beispielsweise verschiedene Fleischerzeugnisse müssen bis zu dem Zeitpunkt des Verzehrs bzw. der Zubereitung durch den Verbraucher kühl, d.h. bei Temperaturen unter 7 °C bis 8 °C, gelagert werden, damit sie genießbar bleiben. Bei dieser Gruppe von Lebens- bzw. Futtermitteln, die somit nur unter Kühlung begrenzt haltbar ist, kann auch bei einer sorgfältigen Herstellungspraxis nicht ausgeschlossen werden, daß über kontaminierte Rohstoffe oder über eine Kontamination einzelner Produktvorstufen oder des Endproduktes für den Verbraucher gefährliche Bakterien in die Lebens- oder Futtermittel gelangen.

Für den Verbraucher im Sinne dieser Erfindung gefährliche Bakterien sind Bakterien, die bakterielle Lebensmittelvergiftungen auslösen können. Zu diesen Bakterien zählen zum einen toxinogene Bakterien, welche bereits im Lebens- oder Futtermittel Toxine bilden können. Der Verzehr des toxinbelasteten Lebens- oder Futtermittels kann zu Toxiinfektionen, d.h zu einer Erkrankung führen. Zum anderen können bakterielle Lebensmittelvergiftungen durch toxiinfektiöse Bakterien ausgelöst werden, die sich im Lebens- oder Futtermittel vermehren können und somit beim Verzehr in den Magen-Darm-Trakt gelangen (G. Seidel und J. Kiesewalter: Bakterielle Lebensmittelinfektionen und -intoxikationen. Berlin: Akademie Verlag, 1992).

Die überwiegende Zahl der von Bakterien hervorgerufenen Lebensmittelvergiftungen tritt nur dann auf, wenn die Erreger relativ hohe Keimdichten in dem verzehrten Lebens- bzw. Futtermittel erreichen (J. Krämer: Lebensmittel-Mikrobiologie. Stuttgart: Ulmer, 1987). Eine Überschreitung der Infektionsdosis (minimale Keimmenge eines Erregers, die zur Auslösung einer Erkrankung notwendig ist) erfolgt bei Lebens- oder Futtermitteln in der Regel dann, wenn eine Unterbrechung der Kühlkette oder die Nichteinhaltung der vorgeschriebenen Kühltemperatur auftreten. Hierbei ist besonders kritisch, daß die Infektionsdosis von den toxinogenen und/oder toxiinfektiösen Bakterien überschritten werden kann, ohne daß es dadurch zu einer sensorischen Veränderung des Lebens- bzw. Futtermittels kommt, die vom Verbraucher wahrgenommen werden könnte. Daher sind Mittel wünschenswert, die bei einer Unterbrechung der Kühlkette oder bei einer Nichteinhaltung der vorgeschriebenen Kühltemperatur das Wachstum toxinogener und/oder toxiinfektiöser Bakterien in den Lebens- oder Futtermitteln hemmen können.

Eine für den Verbraucher befriedigende Hemmung des Wachstums, d.h. Reduzierung der Wachstumsrate dieser gefährlichen Bakterien ist im Stand der Technik trotz einer Vielzahl von Vorschlägen bisher nicht gelungen.

Zwar lässt sich heute bei fast allen Nahrungsmitteln das Wachstum toxinogener und/oder toxiinfektiöser Bakterien durch eine Vielzahl an chemischen Zusatzstoffen (siehe EU-Richtlinie Nr. 95/2/ EG vom 20.2.1995) oder durch physikalische Behandlungen (Anwendung von Hitze, UV-Strahlen, ionisierende Strahlen etc.) hemmen. Allerdings wird die Akzeptanz der Verbraucher gegenüber diesen Maßnahmen zunehmend geringer. Gründe dafür sind u.a. im allergienauslösenden Potential vieler Zusatzstoffe zu suchen. Ferner kann nicht ausgeschlossen werden, daß die Konservierungsmittel im Lebensmittel oder in vivo zu toxischen Substanzen metabolisiert werden (H.-G. Classen, P. S. Elias und W. P. Hammes: Toxikologisch-hygienische Beurteilung von Lebensmittelinhalts- und -zusatzstoffen sowie bedenklicher Verunreinigungen; Berlin und Hamburg: Verlag Paul Parey, 1987).

Ein Beispiel hierfür ist die Pökelung von Fleischerzeugnissen, bei der den Lebensmitteln Nitrit in Form von Nitritpökelsalz zugesetzt wird. Toxikologisch sind hierbei weniger die akute Toxizität des Nitrits als die mögliche Bildung von karzinogenen Nitrosaminen im Lebensmittel oder auch in vivo bedenklich (H. Druckery, R. Preussmann, S. Ivankovic, D. Schmähl: Organotrope karzinogene Wirkungen bei 65 verschiedenen N- Nitroso-Verbindungen in BD-Ratten. Krebsforschung 69, 1967, S. 102 ff.). Ein Verzicht oder eine Reduzierung des Nitritzusatzes wäre deshalb äußerst wünschenswert. Dennoch steht im Stand der Technik "außer Frage, daß der völlige Verzicht auf die Verwendung der Pökelsalze - und zwar ohne den Einsatz anderer Konservierungsstoffe, die auch nicht unbedenklich wären - das Risiko eines bakteriellen Verderbs der Waren und somit eine Gesundheitsgefährdung des Verbrauchers vergrößern würde." (K. Hofmann: Nitrat und seine Folgen in Lebensmitteln tierischer Herkunft. ADI-Verbraucherdienst 31, 1986, S. 98.).

Die zunehmende Nachfrage nach "naturbelassenen" und "chemiefreien" Nahrungsmitteln führt dazu, daß bei den nur unter Kühlung für eine begrenzte Lagerzeit haltbaren Lebens- oder Futtermitteln vielfach das Wachstum toxinogener und/oder toxiinfektiöser Bakterien nur noch durch Kühlung unterbunden wird. Wird diese Sicherheitsmaßnahme nicht eingehalten, d.h. die Kühlkette unterbrochen oder die notwendige Kühltemperatur nicht eingehalten, wird der Verbraucher der Gefahr einer Lebensmittelvergiftung ausgesetzt.

Dieser Gefahr könnte durch den Einsatz biologischer Verfahren begegnet werden. Bestimmte Bakterien, u.a. Milchsäurebakterien sind prinzipiell in der Lage, durch die Produktion einer Vielzahl von Stoffwechselprodukten das Wachstum anderer Bakterien zu hemmen (S.E. Lindgren und W.J. Dobrogosz, FEMS Microbiology Reviews 87:149-173 (1990); H. Asperger, Österreichische Milchwirtschaft 41:1-22 (1986) Beilage 1 zu Heft 4).

Ein für den Verbraucher befriedigender Einsatz von Milchsäurebakterien als Schutzkulturen, d.h. zur Hemmung des Wachstums toxinogener und/oder toxiinfektiöser Bakterien ist jedoch nur möglich, wenn die eingesetzten Bakterien die folgenden strengen Anforderungen erfüllen:
1. Grundvoraussetzung ist die gesundheitliche Unbedenklichkeit, d.h. die eingesetzten Bakterien müssen einen GRAS-Status aufweisen (GRAS: Generally Recognised As Safe; siehe Department of Health and Human Services, Food and Drug Administration, USA: "Substances generally recognized as safe; Proposed Rule" (Docket No. 97N-0103), Federal Register Part III; Vol 62, 1997).
2. Die eingesetzten Bakterien dürfen bei Kühltemperatur keine Stoffwechselaktivitäten entfalten, die sich sensorisch für den Verbraucher negativ auswirken können (beispielsweise Säuerung, Beeinflussung der Farbe, etc.).
3. Ferner müssen während der Kühllagerung über den gesamten Zeitraum hinweg genügend potentiell stoffwechselaktive Bakterien in der Schutzkultur erhalten bleiben, damit die Schutzkultur im Falle einer Temperaturerhöhung auch zum Ende der Lagerfrist in der Lage ist, das Wachstum toxinogener und/oder toxiinfektiöser Bakterien zu hemmen. Unter potentiell stoffwechselaktiven Bakterien werden diejenigen Bakterien verstanden, die bei einer etwaigen Temperaturerhöhung eine Stoffwechselaktivität zeigen.
4. Die eingesetzten Bakterien müssen im Temperaturbereich von mindestens 7 °C bis 8 °C oder darüber das Wachstum von toxinogenen und/oder toxiinfektiösen Bakterien hemmen. Auch bei schnellen Temperaturanstiegen, wie bei einer Unterbrechung der Kühlkette, müssen sie beispielsweise in der Lage sein, die sich unter diesen Bedingungen sehr schnell durchsetzenden Bakterien der Familie *Enterobacteriaceae* (z.B. Salmonellen) zu hemmen. Ferner muß die eingesetzte Schutzkultur in der Lage sein, eine große Bandbreite verschiedener toxinogener und/oder toxiinfektiöser Bakterien zu unterdrücken.

Die bisher im Stand der Technik zur Konservierung von Lebensmitteln eingesetzten Bakterien erfüllen diese Anforderungen nicht.

Bei einem Teil der im Stand der Technik eingesetzten Bakterien beruht die Hemmwirkung auf der Bildung von antagonistischen Proteinen oder Proteinkomplexen, sogenannten Bakteriozinen, die von den Bakterien gezielt zur Unterdrückung bestimmter anderer Bakterien hergestellt werden (Nettles und Barefoot, Journal of Food Protection **6:**338 ff, (1993)). Dabei zeigen Bakteriozine eine antibakterielle Aktivität gegenüber eng verwandten Arten (Tagg et al. (Bacteriological Reviews **40:**722-756, 1976)). Durch die im Stand der Technik bekannten Bakteriozine kann somit nur ein sehr eng begrenztes Spektrum an Bakterien gehemmt werden, das sich größtenteils auf grampositive Bakterien beschränkt (L. de Vuyst und E.J. Vandamme: Bacteriocins of Lactic Acid Bacteria. London, Glasgow, New York, Tokio, Melbourne, Madras; Blackie Academic & Professional, 1994). Der Einsatz bakteriozinbildender Kulturen ist auch deswegen problematisch, weil Bakteriozine häufig eine mangelhafte Stabilität im Lebensmittel aufweisen, im Lebensmittel oft nur in geringen Syntheseraten produziert werden, und weil gegenüber den Bakteriozinen resistente Stämme auftreten können (F.K. Lücke, Deutsche Milchwirtschaft **16:**729 ff, 1994).

Andere im Stand der Technik eingesetzte Bakterien weisen bereits bei Kühllagerung, d.h. unter 7 °C eine Stoffwechselaktivität auf und sind daher als Schutzkultur im Sinne dieser Erfindung nicht einsetzbar (Tanaka, N., et al., Journal of Food Protection **48:**679 ff, (1985); Schmidt, U., Fleischwirtsch., **75:**24 ff, (1995); Collins-Thompson, D.L., et al., Journal of Food Protection **45:**305 ff, (1982); Andersen, L., Fleischwirtsch. **75:**705-712 (1995)). Ferner zeigen diese bekannten und bereits auf ihre Verwendung als Schutzkulturen überprüften Bakterien nur auf wenige toxinogene und/oder toxiinfektiöse Bakterien eine Hemmwirkung und sind daher nicht in der Lage, ein breites Spektrum an toxinogener und/oder toxiinfektiöser Bakterien in den Lebens- und Futtermitteln zu hemmen.

Aufgabe der vorliegenden Erfindung ist es daher, Schutzkulturen zur Verfügung zu stellen, welche die oben genannten Kriterien erfüllen können und deren Einsatz daher in Lebens- oder Futtermitteln unbedenklich ist. Ferner ist es Aufgabe der Erfindung, Lebensmittel zur Verfügung zu stellen, welche die Schutzkulturen enthalten.

Zur Lösung der Aufgabe werden neuartige Schutzkulturen vorgeschlagen, die zur Behandlung von Lebens- oder Futtermitteln dienen sollen. Die erfindungsgemäßen Schutzkulturen umfassen nicht-pathogene, Milchsäure-produzierende Bakterien (Milchsäurebakterien), welche die überraschenden Eigenschaften aufweisen, daß sie bei Einhaltung der Kühltemperatur keine Stoffwechselaktivität zeigen, bei einer Unterbrechung der Kühlkette oder bei Nichteinhaltung der Kühltemperatur hingegen durch die Produktion von Stoffwechselprodukten das Wachstum einer Vielzahl toxinogener und/oder toxiinfektiöser Bakterien hemmen können. Außerdem bleibt die Anzahl der Milchsäurebakterien über die Lagerzeit hinweg in den Lebens- oder Futtermitteln annähernd konstant, so daß auch zum Ende der Lagerung hin eine Hemmung des Wachstums der gefährlichen Bakterien möglich ist. Durch den Einsatz der erfindungsgemäßen Schutzkulturen kann somit der Verbraucher vor einer Vielzahl von toxinogenen und/oder toxiinfektiösen Bakterien wie beispielsweise Salmonellen geschützt werden.

Demgemäß betrifft die Erfindung Schutzkulturen zum Konservieren von Lebens- oder Futtermitteln, die unter Kühlung begrenzt haltbar sind, wobei die Schutzkulturen dadurch gekennzeichnet sind, daß sie nicht-pathogene Milchsäurebakterien mit den folgenden Eigenschaften umfassen:
a) Die Milchsäurebakterien zeigen bei Temperaturen unterhalb von 7 °C bis 8 °C keine sensorisch erfaßbare Stoffwechselaktivität;
b) Die Anzahl der Milchsäurebakterien, welche potentiell stoffwechselaktiv sind, nimmt bei Temperaturen unterhalb von 7 °C bis 8 °C über einen Zeitraum von einer bis zwei Wochen um weniger als zwei Zehnerpotenzen ab; und
c) Die Milchsäurebakterien hemmen bei Temperaturen von mindestens 7 °C bis 8 °C das Wachstum von toxinogenen und/oder toxiinfektiösen Bakterien.

Gemäß einer bevorzugten Ausführungsform umfassen die Schutzkulturen Milchsäurebakterien der Gattungen *Lactococcus, Pediococcus, Lactobacillus, Leuconostoc, Weissella, Bifidobacterium, Enterococcus* und/oder *Sporolactobacillus* oder Mischungen derselben. Gemäß einer besonders bevorzugten Ausführungsform umfassen die Schutzkulturen Milchsäurebakterien der Gattungen Lactococ*cus, Pediococcus, Lactobacillus, Leuconostoc, Enterococcus* oder *Weissella* oder Mischungen derselben, und gemäß einer ganz besonders bevorzugten Ausführungsform Milchsäurebakterien der Gattung *Lactococcus*.

Gemäß einer weiteren bevorzugten Ausführungsform umfassen die Schutzkulturen Milchsäurebakterien der Arten *Lactococcus lactis, Lactococcus garieae, Lactococcus piscium, Lactococcus plantarum* und/oder *Lactococcus raffinolactis* oder Mischungen derselben.

Gemäß einer besonders bevorzugten Ausführungsform umfassen die Schutzkulturen Milchsäurebakterien der Art *Lactococcus lactis*.

Gemäß einer weiteren bevorzugten Ausführungsform umfassen die Schutzkulturen Milchsäurebakterien der Subspezies *Lactococcus lactis subsp. cremoris,* und/oder *Lactococcus lactis subsp. lactis* oder Mischungen derselben. Gemäß einer besonders bevorzugten Ausführungsform umfassen die Schutzkulturen Milchsäurebakterien der Subspezies *Lactococcus lactis subsp. lactis*.

Gemäß einer weiteren bevorzugten Ausführungsform umfassen die Schutzkulturen Milchsäurebakterien der Variante *Lactococcus lactis subsp. lactis var. diacetylactis*.

Gemäß einer ganz besonders bevorzugten Ausführungsform umfassen die Schutzkulturen Milchsäurebakterien des Stammes *Lactococcus lactis subsp. lactis* 1526. Der Stamm *Lactococcus lactis subsp. lactis* 1526 wurde bei der DSMZ-Deutsche Sammlung von Mikroorganismen und Zellkulturen GmbH, Mascheroder Weg 1b, D 38124 Braunschweig, am 21. September 1998 unter der Nummer DSM 12415 hinterlegt.

Gemäß einer besonders bevorzugten Ausführungsform umfassen die erfindungsgemäßen Schutzkulturen eine Mischung der verschiedenen oben genannten Gattungen, Arten, Subspezies und/oder Stämme.

Gemäß einer bevorzugten Ausführungsform zählen zu den Lebensoder Futtermitteln im Sinne dieser Erfindung auch Vorstufen wie Schlachtkörper oder Rohstoffe, die bei der Herstellung der Lebens- oder Futtermittel Verwendung finden.

Gemäß besonders bevorzugten Ausführungsformen sind die Lebensoder Futtermittel Fleisch und Fleischerzeugnisse, Fisch und Fischerzeugnisse, Feinkostsalate sowie Gerichte, die vorgegart gelagert und/oder verkauft werden. Zu diesen Lebens- oder Futtermitteln zählen insbesondere die weiter unten beschriebenen Lebensmittel.

Die Milchsäurebakterien der erfindungsgemäßen Schutzkulturen zeigen bei Temperaturen unterhalb von 7 °C bis 8 °C keine sensorisch erfaßbare Stoffwechselaktivität. Unter sensorisch erfaßbarer Stoffwechselaktivität wird jede Stoffwechselaktivität verstanden, die vom Verbraucher wahrgenommen werden kann. Insbesondere zeigen die Milchsäurebakterien keine Wachstumsaktivität, keine Säuerungsaktivität, und sie produzieren keine Stoffwechselprodukte, welche die Farbe der Lebens- oder Futtermittel beeinflussen können.

Vorzugsweise nimmt die Anzahl der vermehrungsfähigen Milchsäurebakterien bei Temperaturen unterhalb von 7 °C bis 8 °C über einen Zeitraum von ein bis zwei Wochen um weniger als eine Zehnerpotenz, besonders bevorzugt um weniger als eine halbe Zehnerpotenz ab.

Die Milchsäurebakterien der erfindungsgemäßen Schutzkulturen hemmen bei Temperaturen von mindestens 7 °C bis 8 °C das Wachstum der folgenden toxinogenen und/oder toxiinfektiösen Bakterien, wobei die Erfindung nicht auf die Hemmung dieser Bakterien beschränkt ist: *Staphylococcus aureus,* Salmonellen, humanpathogene E. coli Stämme (EIEC, ETEC, EPEC, EHEC), *Shigella, Pseudomonas aeruginosa, Vibrio parahaemolyticus, Aeromonas hydrophila, Camphylobacter jejuni, Bacillus cereus, Clostridium perfringens* und *Clostridium botulinum*.

Gemäß einer besonders bevorzugten Ausführungsform wird das Wachstum der toxinogenen und/oder toxiinfektiösen Bakterien derart gehemmt, daß ihre Anzahl innerhalb von 48 h um nicht mehr als zwei Zehnerpotenzen, vorzugsweise um nicht mehr als eine Zehnerpotenz zunimmt.

Die Milchsäurebakterien der erfindungsgemäßen Schutzkulturen hemmen das Wachstum toxinogener und/oder toxiinfektiöser Bakterien unter anderem durch die Produktion der folgenden antimikrobiell wirksamen Stoffe, wobei erfindungsgemäß eingeschlossen ist, daß die Milchsäurebakterien einige oder alle dieser Stoffe produzieren können und daß das Hemmpotential der Milchsäurebakterien auch auf anderen Stoffen beruhen kann: organische Säuren (Milchsäure, Essigsäure, Ameisensäure, Benzoesäure), Diacetyl, Kohlendioxid, reduzierende Stoffe, die zu einer Senkung des Redoxpotentials führen, Wasserstoffperoxid, Bakteriozine.

Gemäß einer bevorzugten Ausführungsform hemmen die Milchsäurebakterien das Wachstum von toxinogenen und/oder toxiinfektiösen Bakterien durch die Produktion organischer Säuren, vorzugsweise Milchsäure.

Gemäß einer besonders bevorzugten Ausführungsform zeigen die Milchsäurebakterien der erfindungsgemäßen Schutzkulturen bei Temperaturen von mindestens 7 °C bis 8 °C oder darüber eine sensorisch erfaßbare Stoffwechselaktivität, vorzugsweise eine Ansäuerung des Lebens- oder Futtermittels.

Gemäß einer ganz besonders bevorzugten Ausführungsform beträgt die Temperatur, unterhalb derer keine Stoffwechselaktivität der Milchsäurebakterien der erfindungsgemäßen Schutzkulturen vorliegt, und oberhalb derer die Milchsäurebakterien das Wachstum toxinogener und/oder toxiinfektiöser Bakterien hemmen können, 7 °C.

Die Milchsäurebakterien der erfindungsgemäßen Schutzkulturen sind durch das folgende Verfahren erhältlich:
1. Zunächst werden Milchsäurebakterien isoliert, die einer der oben genannten Gattungen, Arten und/oder Stämme oder Mischungen derselben zuzuordnen sind. Die Isolierung geschieht vorzugsweise aus den Lebens- oder Futtermitteln, in denen die Milchsäurebakterien später eingesetzt werden sollen. Auf diese Weise wird eine optimale Anpassung an das Lebens- oder Futtermittel beispielsweise bezüglich Wachstum, Nährstoffausnutzung oder Milchsäureproduktion sichergestellt.
   Verfahren zur Isolierung von Milchsäurebakterien sind im Stand der Technik bekannt (J. Baumgart, W. in Heeschen (Hrsg.): Handbuch Lebensmittelhygiene. Hamburg: Behr's, 1 ff, (1994), insbesondere Kapitel 4, Seite 216 ff) Dort sind ebenfalls Verfahren beschrieben, mittels derer untersucht werden kann, ob es sich bei den isolierten Bakterien tatsächlich um Milchsäurebakterien handelt.
2. Anschließend wird das Wachstumsverhalten sowie die Stoffwechselaktivität bei einer Temperatur unterhalb von 7 °C bis 8 °C sowie darüber untersucht. Unterhalb von 7 °C bis 8 °C dürfen die Bakterien keine sensorisch erfaßbare Stoffwechselaktivität, insbesondere keine Säuerungsaktivität zeigen. Bei Temperaturen von mindestens 7 °C bis 8 °C oder darüber müssen die Bakterien eine sensorisch erfaßbare Stoffwechselaktivität, insbesondere eine Säuerungsaktivität zeigen. Ferner wird untersucht, ob die Anzahl der vermehrungsfähigen isolierten Milchsäurebakterien in einem Lebens- oder Futtermittel, dem diese Milchsäurebakterien zugesetzt wurden, über einen Zeitraum von einer bis zwei Wochen bei Temperaturen unterhalb von 7 °C bis 8 °C um weniger als zwei Zehnerpotenzen abnimmt. Dazu wird eine bestimmte Menge an Bakterien, vorzugsweise 10⁷ Bakterien/cm² Oberfläche des Lebens- oder Futtermittels, zusammen mit dem Lebens- oder Futtermittel für ein bis zwei Wochen inkubiert. Anschließend wird die Anzahl der Bakterien nach für den Fachmann bekannten Methoden bestimmt (J. Baumgart, a.a.O., Kapitel 5, S. 74 ff).
   An diese Charakterisierung kann sich vorzugsweise eine genaue Charakterisierung der isolierten Milchsäurebakterien anschließen. Hierzu stehen eine ganze Reihe von Methoden, wie z.B. eine biochemische Identifizierung mit der miniaturisierten Bunten Reihe "api 50 CHL" (bioMerieux, Marcy-I'Etoile, Frankreich) oder eine Analyse der 16S rDNA Sequenzen, zur Verfügung (B. Pot et al.: Modern methods used for identification and classification of lactic acid bacteria; In: L. de Vuyst und E.J. Vandamme: Bacteriocins of Lactic Acid Bacteria. London, Glasgow, New York, Tokio, Melbourne, Madras: Blackie Academie & Professional 1991; Kapitel 2.3, , S. 40 ff).
3. Anschließend muß geprüft werden, ob die isolierten Milchsäurebakterien in der Lage sind, das Wachstum von toxinogenen und/oder toxiinfektiösen Keimen in Lebens- oder Futtermitteln zu hemmen. Zu diesem Zweck werden die toxinogenen und/oder toxiinfektiösen Bakterien in den Lebens- oder Futtermitteln zusammen mit den isolierten Milchsäurebakterien inkubiert (siehe auch Beispiel 8). Vorzugsweise wird zunächst bei Temperaturen unter 7 °C bis 8 °C und anschließend dann bei Temperaturen von mindestens 7 °C bis 8 °C oder darüber inkubiert. Eine Wachstumshemmung ergibt sich, wenn bei der Inkubation von toxinogenen und/oder toxiinfektiösen Bakterien zusammen mit den isolierten Milchsäurebakterien die toxinogenen und/oder toxiinfektiösen Bakterien langsamer wachsen als bei der Inkubation von toxinogenen und/oder toxiinfektiösen Bakterien ohne Zugabe der isolierten Milchsäurebakterien.

Gegenstand der Erfindung sind ferner Milchsäurebakterien, die zum Stamm *Lactococcus lactis subsp*. *lactis* 1526 (DSM 12415, siehe oben) gehören. Dieser Stamm hat die überraschende Eigenschaft, daß er bei Temperaturen unterhalb von 7 °C bis 8 °C keine sensorisch erfaßbare Stoffwechselaktivität zeigt, hingegen bei Temperaturen von mindestens 7 °C bis 8 °C das Wachstum von toxinogenen und/oder toxiinfektiösen Bakterien hemmen kann. Ferner weist er die überraschende Eigenschaft auf, daß die Anzahl der potentiell stoffwechselaktiven Milchsäurebakterien des Stammes in dem jeweiligen Lebens- oder Futtermittel bei Temperaturen unterhalb von 7 °C bis 8 °C über einen Zeitraum von ein bis zwei Wochen um weniger zwei Zehnerpotenzen abnimmt.

Gegenstand der Erfindung ist ferner die Verwendung der erfindungsgemäßen Schutzkulturen zur Konservierung von Lebens- und Futtermitteln, die unter Kühlung begrenzt haltbar sind. Dabei werden die Lebens- oder Futtermittel mit den erfindungsgemäßen Schutzkulturen behandelt, indem die Lebens- oder Futtermittel mit den Schutzkulturen derart in Kontakt gebracht werden, daß die Schutzkulturen in der Lage sind, das Wachstum von toxinogenen und/oder toxiinfektiösen Bakterien zu hemmen.

Gemäß einer bevorzugten Ausführungsform werden die Lebens- oder Futtermittel mit den erfindungsgemäßen Schutzkulturen behandelt, indem die Schutzkulturen auf die Oberfläche der Erzeugnisse aufgebracht werden, vorzugsweise durch Besprühen oder Einreiben. Nach einer weiteren Ausführungsform werden die Erzeugnisse mit den Schutzkulturen behandelt, indem die Schutzkulturen in das Erzeugnis eingemischt und/oder eingerührt werden.

Gemäß einer bevorzugten Ausführungform werden die Lebens- oder Futtermittel mit einem Pulver behandelt, das die Schutzkulturen und, gegebenenfalls, geeignete Trägerstoffe umfaßt. Geeignete Trägerstoffe umfassen beispielsweise Saccharide, vorzugsweise Mono- oder Disaccharide.

Gemäß einer weiteren bevorzugten Ausführungsform werden die Lebens- oder Futtermittel mit einem flüssigen Medium behandelt, das die Schutzkulturen umfaßt. Das flüssige Medium muß derart gestaltet sein, daß es die Überlebensfähigkeit der Kultur während der Behandlung garantiert.

Gemäß einer besonders bevorzugten Ausführungsform ist das flüssige Medium ein wäßriges Medium wie physiologische Kochsalzlösung oder Trinkwasser.

Gemäß einer bevorzugten Ausführungsform werden die Lebens- oder Futtermittel mit einer Milchsäurebakterienmenge von 10⁴ bis 10⁸ Milchsäurebakterien pro g oder ml oder cm² Oberfläche des Lebensoder Futtermittels behandelt, vorzugsweise mit einer Milchsäurebakterienmenge von 10⁵ bis 10⁶ Milchsäurebakterien pro g oder ml oder cm² Oberfläche.

Vorzugsweise wird bei der Behandlung den Lebens- oder Futtermitteln außerdem eine Kohlenstoffquelle, vorzugsweise Kohlenhydrate, besonders bevorzugt Glucose, Saccharose, Lactose zugegeben.

Gemäß einer bevorzugten Ausführungsform stellen die erfindungsgemäßen Schutzkulturen, mit denen die Lebens- oder Futtermittel behandelt werden, eine Mischung der oben genannten Gattungen, Arten, Subspezies und/oder Stämme dar.

Gegenstand der Erfindung ist ferner ein Lebens- oder Futtermittel, das dadurch gekennzeichnet ist, daß es die erfindungsgemäßen Schutzkulturen enthält. Dazu wird das Lebens- oder Futtermittel mit den erfindungsgemäßen Schutzkulturen gemäß den oben dargestellten Ausführungsformen behandelt.

Gemäß einer bevorzugten Ausführungsform ist das Lebens- oder Futtermittel ausgewählt aus der Gruppe der Fleisch- oder Fleischerzeugnisse, ganz besonders bevorzugt aus der Gruppe bestehend aus:
- portioniertem Frischfleisch, Innereien, Geflügel und Geflügelstücke, die durch Aufbringen der erfindungsgemäßen Schutzkulturen auf die Oberfläche behandelt sind;
- Schlachtkörpern, Teilstücken, entbeinten Teilstücken, Aufschnittware, wie beispielsweise Brühwurstaufschnitt, Kochwurstaufschnitt, Aufschnitt von Koch- oder Rohpökelwaren, die durch Aufbringen der erfindungsgemäßen Schutzkulturen behandelt sind;
- zerkleinerten Fleischerzeugnissen wie beispielsweise Hackfleisch (Schabefleisch, Beefsteakhack, Tartar, Rinderhackfleisch, Schweinehackfleisch, Schweinemett), Hackfleischzubereitungen, Bratwursterzeugnissen, die durch Einrühren oder durch Aufbringen der erfindungsgemäßen Schutzkulturen auf die Oberfläche behandelt sind.

Nach einer weiteren besonders bevorzugten Ausführungsform ist das Lebens- oder Futtermittel ausgewählt aus der Gruppe der Fisch und Fischerzeugnissen, ganz besonders bevorzugt aus der Gruppe bestehend aus Frischfisch (Fischfilet, Fischkoteletts), Räucherfisch wie Aufschnittware, oder Weich- und Krustentieren, die jeweils durch Aufbringen der erfindungsgemäßen Schutzkulturen auf die Oberfläche behandelt sind.

Nach einer weiteren besonders bevorzugten Ausführungsform sind die Lebensmittel Feinkostsalate. Besonders bevorzugt werden die Lebensmittel aus der Gruppe bestehend aus Salaten auf Fleisch-, Fisch-, Weichtier-, Krustentier- und Gemüsegrundlage oder Salaten mit Teigwaren ausgewählt, wobei nach einer besonders bevorzugten Ausführungsform die Feinkostsalate auf Mayonnaise, Salatmayonnaise- oder Remouladenbasis hergestellt werden, wobei jeweils die Feinkostsalate durch Einmischen/Einrühren der erfindungsgemäßen Schutzkulturen oder durch Aufbringen der erfindungsgemäßen Schutzkulturen auf die Oberfläche behandelt werden.

Nach einer weiteren besonders bevorzugten Ausführungsform sind die Lebensmittel vorgegarte Fertiggerichte. Vorgegarte Fertiggerichte im Sinne dieser Erfindung sind Gerichte, die gegart aufbewahrt und/oder verkauft werden. Diese Gerichte werden durch Einmischen/Einrühren der erfindungsgemäßen Schutzkulturen oder durch Aufbringen der erfindungsgemäßen Schutzkulturen auf die Oberfläche behandelt.

Nach einer weiteren besonders bevorzugten Ausführungsform sind die Lebensmittel ausgewählt aus der Gruppe der Milchprodukte, ganz besonders bevorzugt aus der Gruppe bestehend aus Frischmilch, Joghurt, Quark und Käse.

Die vorliegende Erfindung stellt somit zum erstmals Schutzkulturen zur Verfügung, die bei Temperaturen von mindestens 7 °C bis 8 °C oder darüber das Wachstum von toxinogenen und/oder toxiinfektiösen Bakterien hemmen, bei Temperaturen unter 7 °C bis 8 °C hingegen keine sensorisch erfaßbare Stoffwechselaktivität zeigen. Die erfindungsgemäßen Schutzkulturen können direkt in Lebens- oder Futtermitteln, die für den Einsatz beim Menschen oder Tieren bestimmt sind und die nur unter Kühlung begrenzt haltbar sind, eingesetzt werden, da sie für den Verbraucher nicht pathogen sind.

Im folgenden wird die Erfindung durch Figuren, Tabellen und Beispiele erläutert.

### Beschreibung der Figuren

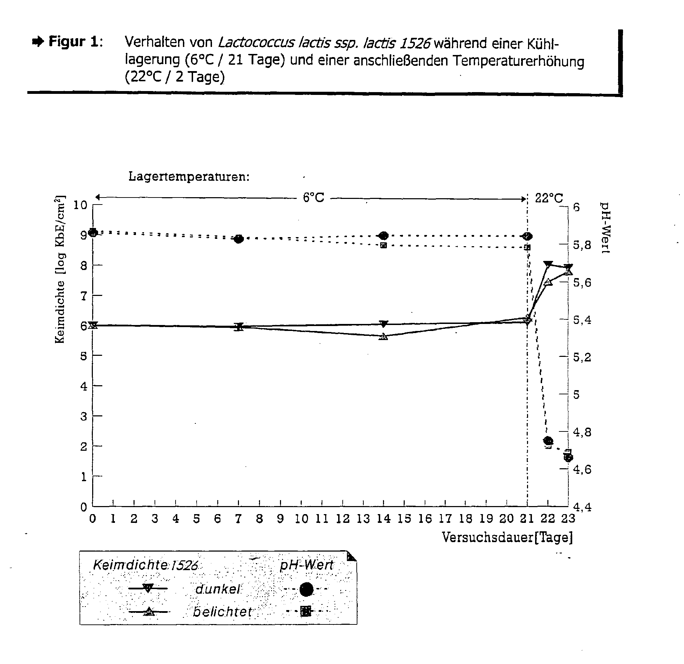

- Figur 1: Verhalten von *Lactococcus lactis subsp. lactis 1526* in vakuumverpackten Brühwurstaufschnitten während einer 3wöchigen Kühllagerung (6 °C) und einer anschließend simulierten Kühlkettenunterbrechung (48 h/22 °C). Auf der linken Y-Achse ist die Keimdichte (log KbE/cm²) an *L. lactis subsp. lactis 1526* aufgetragen, während auf der rechten y- Achse der pH- Wert in der Brühwurst während des Versuches aufgetragen ist. Auf der der X-Achse ist die Versuchsdauer aufgetragen. Der Brühwurstaufschnitt wurde vakuumverpackt, die Deckfolie bestand aus OPP/SIOₓ/PE. Im Falle der belichteten Proben wurden die Proben bei 1300 Lux belichtet, wobei der Abstand zwischen vakuumverpackten Proben und Leuchtstoffröhren 40 cm betrug.

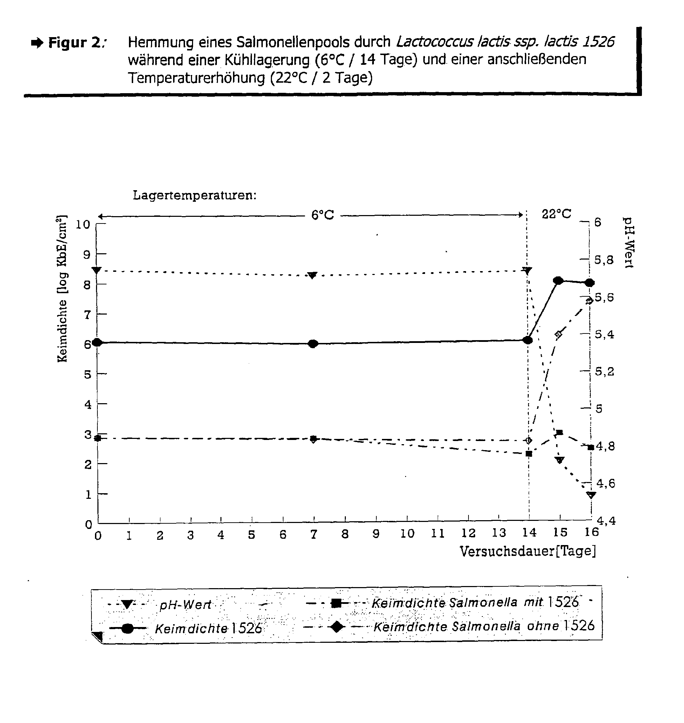

- Figur 2: Hemmung des Salmonellen-Pools während einer simulierten Kühlkettenunterbrechung (22 °C/48 h) durch *L. lactis subsp.lactis 1526*. Auf der Y-Achse ist die Keimdichte der Bakterien aufgetragen, während auf der X- Achse die Versuchsdauer aufgetragen ist.

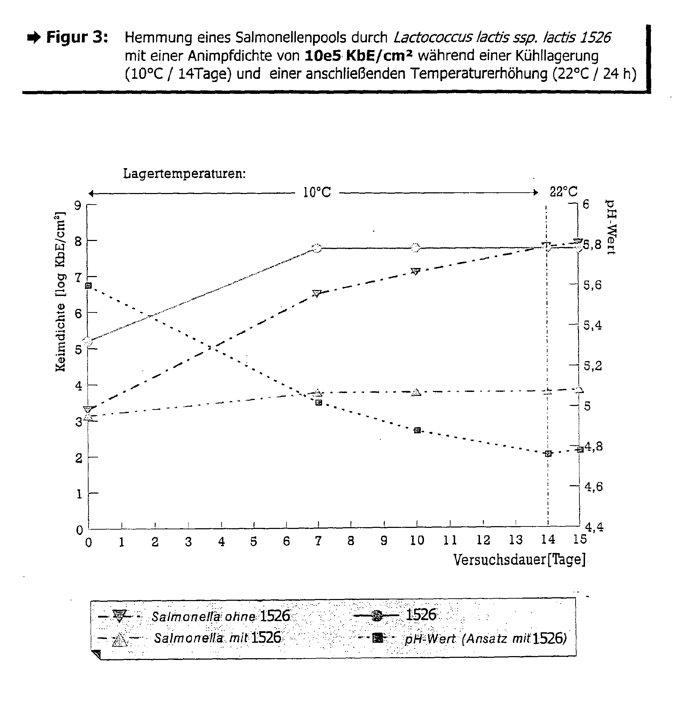

- Figur 3: Hemmung des Salmonellen-Pools während einer nichtsachgerechten Kühllagerung (10 °C) und eine anschließenden Kühlkettenunterbrechung (22 °C) durch *Lactococcus lactis subsp. lactis 1526*. Auf der Y-Achse ist die Keimdichte der Bakterien aufgetragen, während auf der X-Achse die Versuchsdauer aufgetragen ist.

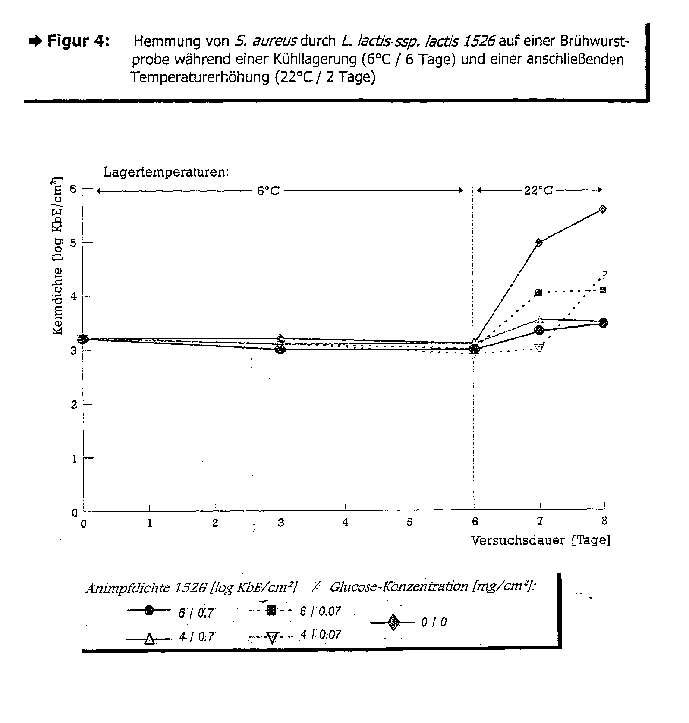

- Figur 4: Hemmung von *Staphylococcus aureus subsp. aureus* während einer simulierten Kühlkettenunterbrechung (22 °C/48 h) durch *Lactococcus lactis subsp. lactis 1526*. Die Hemmaktivität ist in Abhängigkeit zur Animpfdichte der Schutzkultur sowie zu der zugesetzten Glucosemenge angegeben. Die Y-Achse bezeichnet die Keimdichte der Staphylokokken, während an der X-Achse die Versuchsdauer angezeigt ist.

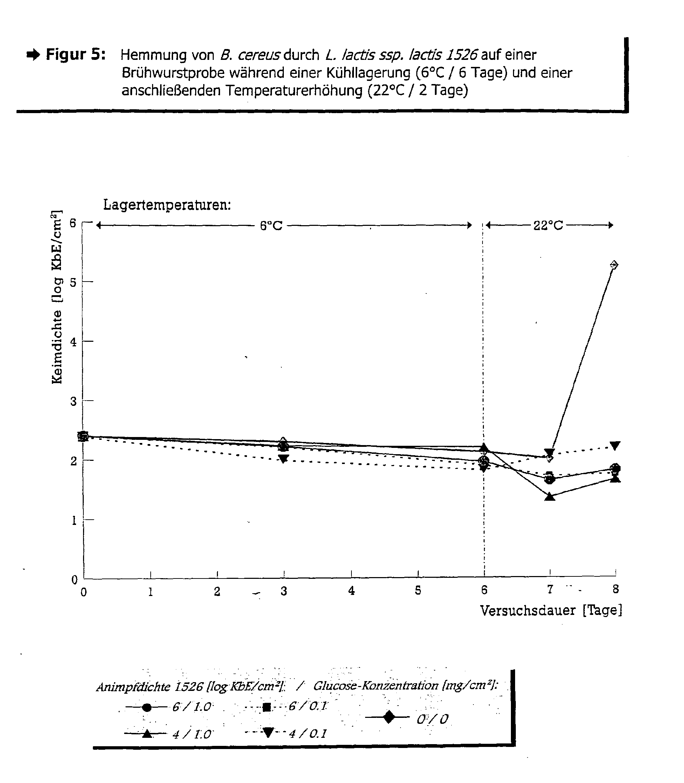

- Figur 5: Hemmung von *Bacillus cereus* während einer simulierten Kühlkettenunterbrechung (22 °C/48 h) durch *L. lactis subsp. lactis 1526*. Die Hemmaktivität ist in Abhängigkeit zu Animpfdichte der Schutzkultur sowie zu der zugesetzten Glucosemenge angegeben. Die Y-Achse bezeichnet die Keimdichte von *Bacillus cereus,* während an der X-Achse die Versuchsdauer angezeigt ist.

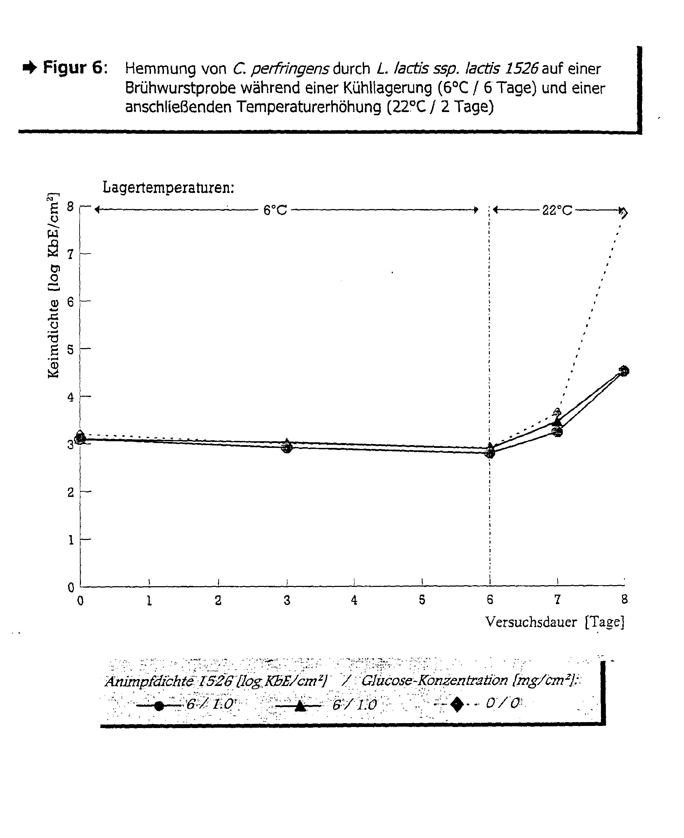

- Figur 6: Hemmung von *Clostridium perfringens* während einer simulierten Kühlkettenunterbrechung (22 °C/48 h) durch *L. lactis subsp. lactis 1526*. Die Hemmaktivität ist in Abhängigkeit zu Animpfdichte der Schutzkultür sowie zu der zugesetzten Glucosemenge angegeben. Die Y-Achse bezeichnet die Keimdichte der Chlostridien, während an der X-Achse die Versuchsdauer angezeigt ist.

### Beispiele

### Beispiel 1

### Isolierung von Milchsäurebakterienstämmen aus Lebensmittel

Zur Anlegung einer Stammsammlung wurden Milchsäurebakterien vorwiegend aus Fleischerzeugnissen isoliert. Dies sollte gewährleisten, daß es sich bei den isolierten Milchsäurebakterien um Organismen handelt, die an das Substrat "Fleisch" adaptiert sind. Weiterhin wurde gefordert, daß sich die Isolate gegenüber einer konkurrierenden Flora durchsetzen können. Deshalb wurden die Fleischerzeugnisse vor der Isolierung bei 30 °C für 48 h gelagert. Somit konnte sich die Milchsäurebakterien-Flora durchsetzen, die dominierenden Milchsäurebakterien erreichten in den Proben hohe Keimdichten.

Bei lose verpackten Proben, die neben vakuumverpackten Erzeugnissen herangezogen wurden, wurden die Keime ausschließlich aus Teilen isoliert, die aus dem Inneren der Probenstücke steril herausgetrennt wurden.

Die ca. 5 bis 10 g schweren Probestücke wurden in 90 ml sterile Ringerlösung (Unipath GmBH, D-46467 Wesel, BR 52) eingebracht und entsprechend ihrer Beschaffenheit mit einem Ultra-Turrax (T 25, Fa. JANKE & KUNKEL/ Staufen) für zwei Minuten bei 13500 U/min oder im Stomacher (LabBlender 400, SEWARD-MEDICAL/London) für zehn Minuten zerkleinert und suspendiert. Von den somit gewonnenen Suspensionen wurden fortlaufende Verdünnungsreihen in jeweils 9 ml steriler Ringerlösung hergestellt. Von den Verdünnungsstufen 10⁻⁴ bis 10⁻⁸ wurden jeweils 100 µl auf Chalmers (modifiziert)- sowie auf MRS-Nährböden ausgespatelt (zu den Nährböden siehe Tabelle 2). Die Inkubation erfolgte bei 30 °C in Anaerobiertöpfen (Unipath, HP 11), in denen unter Verwendung von AnaeroGen (Unipath, AN 35) eine anaerobe Atmosphäre erzeugt wurde.

Nach 3 bis 4 Tagen wurden von den Nährböden pro Erzeugnis-maximal fünf Kolonien abgenommen, die sich aufgrund ihrer makroskopischen Morphologie unterschieden. Bei den Chalmers-Nährböden wurden vor allem jene Kolonien berücksichtigt, die sich durch einen großen Hofdurchmesser (entfärbt, klar) auszeichneten. Dies kann als Indiz für eine starke Säurebildung gewertet werden. Die abgenommenen Kulturen wurden in 5 ml einer MRS-Bouillon suspendiert und für 48 Stunden bei 30 °C anaerob bebrütet. Wenn sich nach der zweitägigen Inkubation eine deutliche Trübung zeigte, wurde auf Chalmers und MRS ein Verdünnungsausstrich angelegt. Nach einer entsprechenden Bebrütung (4 Tage/30 °C/anaerob) wurden Einzelkolonien abgenommen und die so gewonnene Reinkultur erneut in einer MRS-Bouillon angezüchtet. Die Isolate wurden als Gefrierpräparate in die Stammsammlung aufgenommen. Zur Herstellung eines Präparates wurden die frisch angezüchteten Reinkulturen auf einen MRS-Nährboden ausgespatelt und entsprechend den bereits aufgeführten Bedingungen inkubiert. Nach 4 bis 5 Tagen konnte der Bakterienrasen mit 1,5 ml einer Caseinpepton-Sojamehlpepton-Lösung USP (Unipath, CM 129) abgeschwemmt und in 6 ml einer sterilen Magermilchlösung (Unipath, L 31) eingebracht werden. Nach dem Vermischen wurde die Bakteriensuspension in Kulturröhrchen abgefüllt und bei -20 °C gefriergelagert.

### Beispiel 2

### Klassifizierung der islierten Milchsäurebakterienstämme aufgrund genereller Merkmale

Die in die Stammsammlung aufgenommenen Isolate wurden aufgrund der Gram-Reaktion, des Katalase- sowie des Oxidations-Fermentationstests hinsichtlich ihres Wachstumsverhaltens auf entsprechenden Nährmedien (MRS, Chalmers, Rogosa) als auch aufgrund einer mikroskopischen und makroskopischen Beurteilung ihrer Morphologie grob klassifiziert. Hierbei gelten eine positive Gram-Reaktion, ein negativer Katalase-Test, ein fermentativer Kohlenhydratabbau sowie die Kolonienmorphologie auf einem modifizierten Chalmers-Nährboden (Hofbildung durch Säure) als generelle Merkmale, die eine Zugehörigkeit der Isolate zu der Gruppe der Milchsäurebakterien wahrscheinlich machen (Baumgart, J. et al., a.a.O., Kapitel 4.8, S 245 ff). Eine Gasbildung aus Glucose im OF-Test, das mikroskopische Bild sowie das Wachstumsverhalten auf einem Rogosa-Nährboden können dagegen bereits Hinweise auf eine bestimmte Gattungszugehörigkeit geben (Baumgart, J. et al., a.a.O., Kapitel 4.8, S 245 ff).

### Gram-Reaktion

Die Gram-Färbung wurde an frisch angezüchteten Kulturen (Oberflächen-Kolonien) durchgeführt. Für die Färbung wurde das Testkit ColorGram 2 (bioMérieux / Marcy-l'Etoile, Frankreich) herangezogen. Zur Kontrolle des Färbeverfahrens wurde je eine grampositive (*Bacillus subtilis*) sowie eine gram-negative Kultur (*Escherichia coli*) mitbehandelt.

### Katalase-Test

Oberflächenkolonien des zu testenden Isolates wurden mit einer Impföse abgenommen und auf einem Objektträger mit einer 3%igen H₂O₂-Lösung (Bactident Katalase, Merck, 11351) verrieben. Durch eine Gasbildung wurde das Vorhandensein von Katalase angezeigt.

### Oxidations-Fermentations-Test

Durch den OF-Test nach Hugh, R. and Leifson, E., J. Bact., **66:** 24 ff (1953) wurde überprüft, ob das Bakterien-Isolat Glucose fermentativ unter Säure- und einer evtl. Gasbildung verwertet. Als Testmedium wurde ein kohlenhydratfreier OF-Basis-Nährboden (Merck, 10282) verwendet, dem nach dem Autoklavieren eine 10%ige sterilfiltrierte Glucoselösung (100 ml auf 1 l Nährboden) zugesetzt wurde. Die über eine Impfnadel abgenommene Reinkultur wurde in jeweils zwei Röhrchen, die mit dem Nährboden befüllt waren, parallel im Stichverfahren überimpft. Eines der beiden Röhrchen wurde nach der Ausführung des Impfstiches mit sterilem Paraffinöl fingerbreit überschichtet, um einen Sauerstoffzutritt zu verhindern. Nach einer Inkubation von 2-4 Tagen bei 37 °C konnte im Falle einer fermentativen Glucoseverwertung ein Farbumschlag durch den Indikator Bromthymolblau in beiden Röhrchen beobachtet werden. Weiterhin wurde bei der Auswertung auf eine evtl. Gasbildung (Blasen und/oder Spalten in der Nährbodensäule) sowie auf eine evtl. Beweglichkeit (durchschwärmter Nährboden) geachtet.

### Biochemische Identifizierung der Isolate

Die biochemische Identifizierung der als Milchsäurebakterien klassifizierten Isolate wurde mit der miniaturisierten Bunten Reihe api 50 CHL (bioMérieux, Marcy-l'Etoile, Frankreich) durchgeführt. In dem System api 50 CHL wird der Metabolismus anhand von 49 Kohlenhydraten überprüft. Das sich daraus ergebende biochemische Profil wurde mit Hilfe der dafür zur Verfügung stehenden Software APILAB Plus interpretiert.

Die zu identifizierenden Isolate wurden aus einer Gefrierkultur in einer MRS-Bouillon angezüchtet, abzentrifugiert und anschließend in Ringerlösung aufgenommen. Das für die Versuche benötigte Inokulum konnte mit Hilfe des McFarland Standards 2,0 (bioMérieux, 70900) eingestellt werden. Die Mikroröhrchen der Teststreifen wurden mit 100 µl der Keimsuspension beimpft, mit sterilem Paraffinöl überschichtet und in einer feuchten Kammer bei 30 °C bebrütet. Die Auswertung der Mikroröhrchen erfolgte nach 24 und 48 h, wobei ein durch eine Fermentation bedingter Bromkresolpurpurindikator-Umschlag als positiv gewertet wurde.

### Beispiel 3

### Bestimmung des Wachstums sowie der Säuerungsaktivität der isolierten Milchsäurebakterienstämme

Die Erfassung des Wachstumsverhaltens der Milchsäurebakterien-Isolate wurde in Anlehnung an die Methode von Reuter, G., Archiv für Lebensmittelhygiene, **12:** 257 ff (1970) durchgeführt. Als Nährmedium wurde eine MRS-Bouillon herangezogen, wobei je 10 ml in Reagenzgläser eingebracht wurden. Die Nährlösungen wurden anschließend mit je 10⁵ Keimen beimpft. Die geprüften Temperaturen betrugen 6 °C, 10 °C und 15 °C, wobei sich der Bebrütungszeitraum nach der jeweiligen Temperatur richtete. Die bei 6 °C gelagerten Proben wurden nach 14 Tagen, die 10 °C-Proben nach 7 und die 15 °C-Proben nach 5 Tagen beurteilt. Nach dem zweiwöchigen Versuchszeitraum wurden die bei 6 °C gelagerten Proben für 24 Stunden bei 22 °C bebrütet, um festzustellen, ob die Kultur nach der Kühllagerung noch eine ausreichende Vitalität aufweist.

Nach den entsprechenden Versuchszeiträumen wurde eine Keimvermehrung anhand einer Trübung der Bouillon oder aufgrund eines Bodensatzes festgestellt. Weiterhin wurde der pH-Wert der Nährlösung bestimmt, um die Säuerungsaktivitäten der Isolate zu erfassen. Als positiv wurden die Kulturen bewertet, die bei 6 °C kein Wachstum bzw. keine Säuerung zeigten. Dagegen mußte bei 15 °C eine deutliche Vermehrung auftreten und der pH-Wert der Nährlösung unter dem Wert von 5,0 liegen.

### Beispiel 4

### Charakterisierung der MSB-Kultur L. lactis subsp. lactis 1526

Die Milchsäurebakterten-Kultur mit der Stamm-Bezeichnung *Lactococcus lactis subsp*. *lactis 1526* wurde aus einem vakuumverpacktem Brühwurstaufschnitt, der aus Hongkong bezogen wurde, gemäß Beispielen 1 bis 3 isoliert. Das Oberflächenwachstum der Kultur bei 30 °C unter anaeroben Bedingungen ist auf den Nährböden nach de Man, Rogosa und Sharpe (MRS) sowie auf einem Nährboden nach Chalmers (modifiziert) als gut zu bezeichnen. Auf einem MRS-Nährboden zeigt die Kultur nach einer dreitägigen Inkubation bei 30 °C glatte, runde Kolonien mit einem Hofdurchmesser > 1 mm. Auf einem Nährboden nach Chalmers bilden sich Kolonien, die sich vor allem durch eine ausgesprochen deutliche Hofbildung auszeichnen, was auf eine starke Säurebildung hinweist. Das Wachstum auf einem Rogosa-Nährboden, der auf eine selektive Isolierung von Laktobazillen ausgelegt ist, ist dagegen nur sehr schwach.

Die mikroskopische Morphologie ist bei unterschiedlichen Anzuchtbedingungen sehr uneinheitlich. Die Beschreibung variiert von "kokkoid" bis zu "kurze Stäbchen", wobei beide Formen in einem Präparat auftreten können. Die Keime liegen größtenteils in Ketten, teils auch paarweise vor. Die Kultur zeigt eine ausgesprochen gute Vitalität bei der Anzucht nach der Gefrierkultivierung. In der Anzuchtsbouillon (MRS-Bouillon) wurden nach 24 h bei 30 °C Keimdichten von über 109 KbE/ml erreicht.

Die biochemische Identifizierung mit dem Schnelltest api 50CH von bioMerieux ergab als Taxon der ersten Wahl die Spezies "Lactococcus lactis subsp. lactis" mit einer Prozenridentifizierung von 98,4%. Eine von der Deutschen Sammlung von Mikroorganismen und Zellkulturen GmbH in Braunschweig durchgeführte DNA- Sequenzierung bestätigte die biochemische Identifizierung. Die Untersuchung der 16S rDNA Sequenzähnlichkeit mit der Sequenzierung des Bereiches mit der größten Variabilität ergab eine 99,8%-Übereinstimmung mit Lactococcus lactis subsp. lactis. Das Endprodukt der Glucosevergärung ist Milchsäure mit einer L(+)-Konfiguration. Lactat kann u.a. aus Glucose, Fructose, Mannose und Lactose, sowie aus Ribose, Trehalose und Saccharose gebildet werden.

### Beispiel 5

### Bestimmung von Keimdichten

Die Keimdichten wurden nach der von J. Baumgart, W. in Heeschen, (Hrsg.): Handbuch Lebensmittelhygiene. Hamburg: Behr's, 1 ff, (1994), beschriebenen Methode bestimmt. Die zu testende Probe (20 g) wurde mit 80 ml steriler Ringerlösung in einen Stomacherbeutel auf einer oberschaligen Waage eingewogen. Das Homogenisieren wurde mit einem Beutel-Walk-Gerät (Stomacher 400) durchgeführt. Die Homogenisierungszeit betrug bei mittlerer Laufgeschwindigkeit entgegen der Empfehlung von Baumgart 10 min., da die in der Literatur vorgeschlagenen Homogenisierungszeiten von max. 60 s sich bei dem zu untersuchenden Lebensmittel als unzureichend erwiesen.

Aus der homogenisierten Probe wurde 1 ml entnommen und in 9 ml Ringerlösung pipettiert. Die Anzahl der insgesamt angelegten Dezimalverdünnungen einer Probe richtete sich nach der zu erwartenden Keimzahl. Von den einzelnen Verdünnungsstufen wurden entweder 100 µl für eine Oberflächenkultur oder 1 ml für eine Gußkultur entnommen und nach dem jeweiligen Verfahren auf den entsprechenden Nährböden aufgespatelt oder mit dem noch flüssigen Nährboden vermengt. Die für die einzelnen Keimgruppen eingesetzten Nährmedien sowie die entsprechenden Inkubationsbedingungen sind in Beispielen 6 und 7 aufgeführt.

Für jede Keimzahlbestimmung wurden mind. 2 Proben herangezogen, wobei von jeder Probe zwei Verdünnungsreihen angelegt wurden. Aus den pro Versuchsansatz ermittelten Keimzahlen wurde die Keimdichte durch das arithmetische Mittel bestimmt. Die Wiederholungsversuche wurden zeitlich versetzt durchgeführt, wobei unterschiedliche Probenchargen für die Versuche eingesetzt wurden. Wurde bei den gleichen Versuchsansätzen eine Differenz der Keimdichten von log 1 überschritten (|log KbE _{Versuchl} - log KbE _{Versuch 2}| > 1), mußte der Versuch wiederholt werden.

### Beispiel 6

### Anzucht von Hygienerisikokeimen

Die Anzucht der gefrierkultivierten Hygienerisikokeime erfolgte für 24 h in jeweils 100 ml Caseinpepton-Sojamehlpepton-Lösung USP (Unipath, CM 129). *Bacillus cereus* und *Clostridium botulinum* wurden bei 30 °C, *Staphylococcus aureus, Clostridium perfringens* sowie die einzelnen Salmonellen-Stämme bei 37 °C bebrütet. Die Anzucht von *C. perfringens* erfolgte unter anaeroben Bedingungen.

Die verwendeten Medien, die Methodik sowie die Inkubationsbedingungen zur Keimzahlbestimmung der als Hygienerisikokeime verwendeten Testkeime sind in Tabelle 1 aufgeführt.

**Tabelle 1**

| **Testmikroorganismen** | **Medium** | **Methode** | **Inkubationsbedingungen (Temperatur/Zeit/Atmosphäre)** |
|---|---|---|---|
| | | | |
| *Staphylococcus aurcus* (DSM 346) | Baird-Parker-Nährboden | Gußplatten-Verfahren | 37°C / 24-48h / aerob |
| | | | |
| *Salmonella* ¹⁾ | Gassner-Nähr-boden/Brillantgrün-Phenolrot-Lactose-Saccharose-Agar. mod. | Spatelverfahren | 37°C / 24h / aerob |
| | | | |
| *Bacillus cereus* (DSM 31) | Bacillus-Cereus-Selektiv-Nährboden | Spatelverfahren | 37°C / 24-18h / aerob |
| | | | |
| *Clostridium perfringens* (DSM 756 Typ A) | OPSP-Selektivnährboden | Gußplatten-Verfahren | 37°C / 18-24h / anaerob |

| | | | |
|---|---|---|---|
| ¹⁾ Folgende Salmonellen aus dem Nationalen Referenzzentrum für Salmonellosen wurden verwendet: Salmonella enteritidis (Se 125/94 Lysotyp 8/7. Se 203/94 Lysotyp 8/7. Se 315/94 Lysotyp 4/6). Salmonella panama (SZ 1107/93. SZ 1249/93. SZ 2365/93). Salmonella typhimurium (SZ 218/94. SZ 235/94. SZ 284/94) | | | |

### Beispiel 7

### Anzucht von Milchsäurebakterienstämmen

Die in den Beispielen 1 bis 3 als positiv klassifizierten Milchsäurebakterienstämme wurden zu Versuchsbeginn in je 10 ml MRS-Bouillon (Unipath, CM 359) für 24 h bei 30 °C angezüchtet. Nach einer Kühllagerung bei 6 °C für max. 10 Tage wurde der Anzuchtsbouillon 100 µl Bakteriensuspension entnommen und zur erneuten Anzucht in 10 ml MRS-Nährlösung eingebracht. Nach der 2. Anzucht (30 °C/24 h) wurde die Bakterienmasse für 10 min bei 8.000 U/min abzentrifugiert (Biofuge 28 RS, Fa. HERAEUS SEPA-TECH/ Osterode). Anschließend wurde der Überstand abgegossen und der Bodensatz mit der gleichen Menge an steriler Ringerlösung aufgeschlämmt. Die Zellsuspension wurde nochmals unter gleichen Bedingungen zentrifugiert, der Überstand entfernt und wiederum durch die gleiche Menge an steriler Ringerlösung ersetzt. Die somit gereinigten Kulturen wurden bis zu ihrem Einsatz kühlgelagert (6 °C, max. 7 Tage) und nach Bestimmung der Keimdichte den Brühwurst-Proben zugesetzt.

Zur Bestimmung der Keimdichten bzw. zum Nachweis der Keime wurde ein modifizierter Chalmers-Nährboden herangezogen. Von den entsprechenden Verdünnungen wurden Gußkulturen angelegt, die für 3 - 5 Tage bei 30 °C bebrütet wurden.

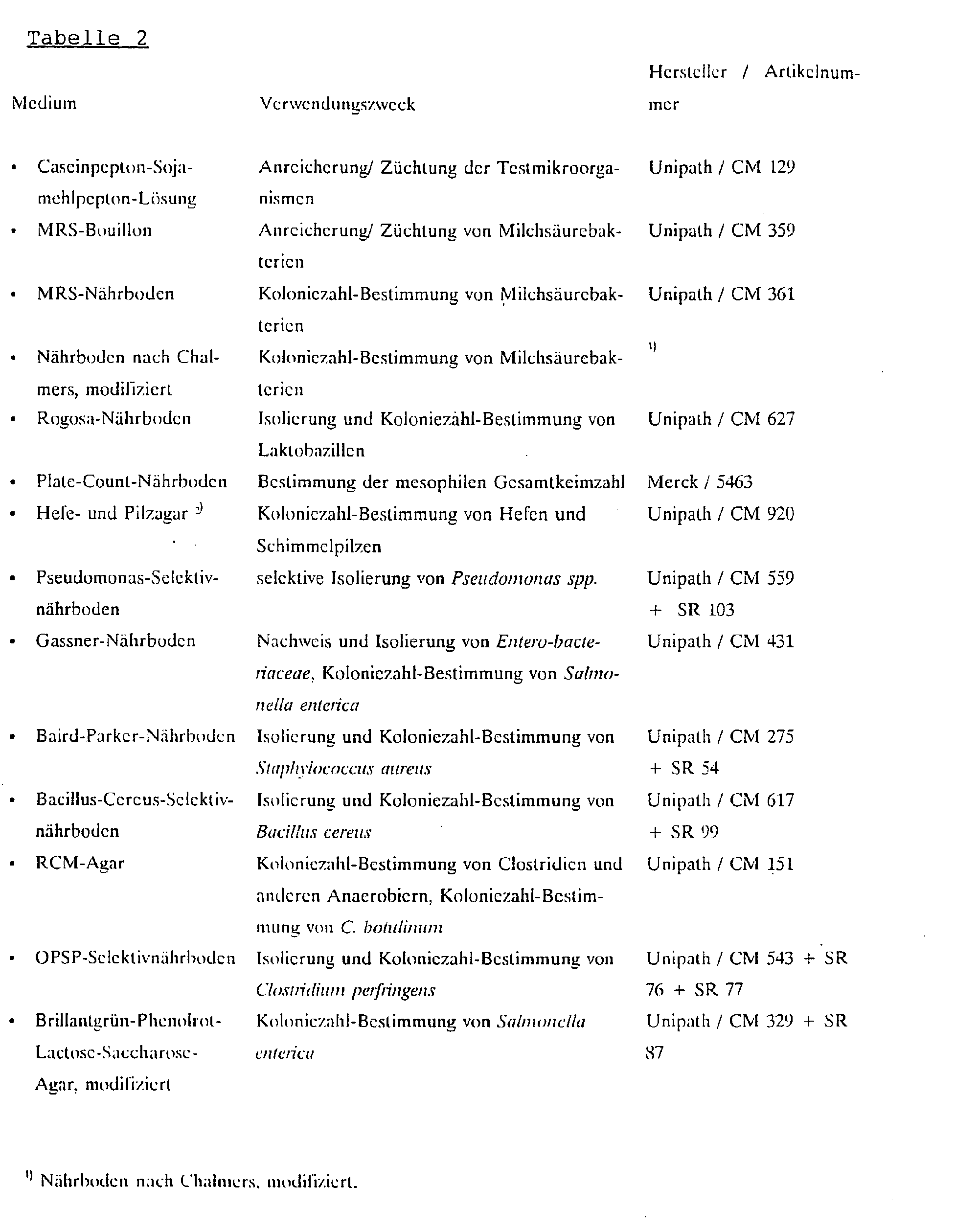

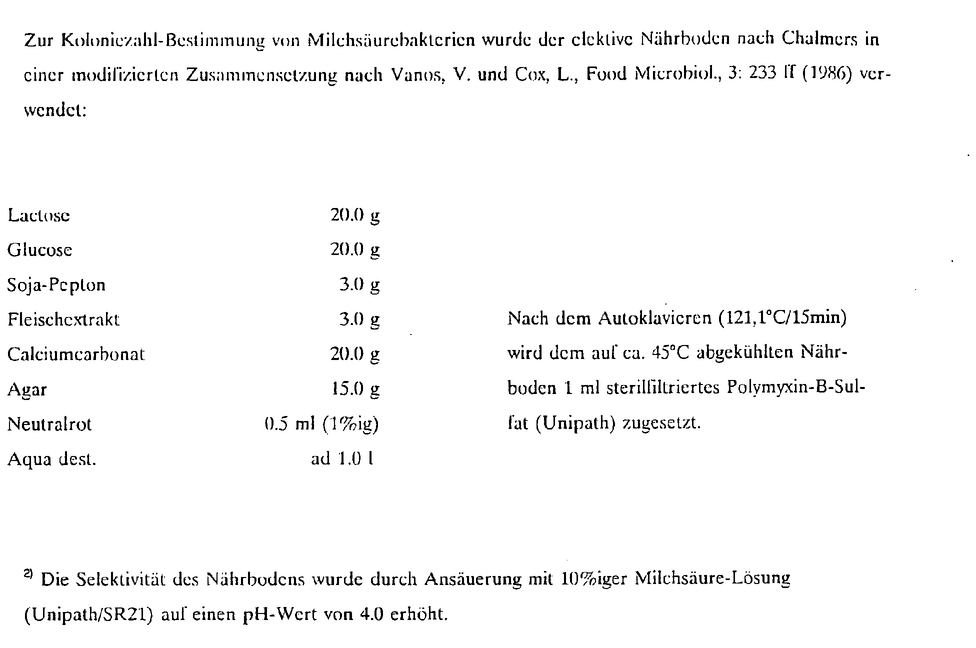

### Beispiel 8

### Hemmung der toxinogenen und/oder toxiinfektösen Keime in Vakuum verpackten Brühwurstaufschnitt durch Lactococcus lacis subsp. lactis 1526

Die wichtigste Anforderung, die an eine Schutzkultur. gestellt werden muß, ist die hygienische Absicherung eines Lebensmittels bei einer nicht sachgerechten Kühllagerung. Somit müssen die eingesetzten erfindungsgemäßen Bakterien gegenüber toxinogenen und/oder toxiinfektiösen Bakterien ausreichend antagonistisch wirksam sein und daher eine Vermehrung und/oder Toxinbildung deutlich unterdrücken. Ergebnis der in den Beispielen 1 bis 4 gezeigten Versuche ist, daß Bakterien, die zum Stamm *Lactococcus lactis subsp. lactis 1526* gehören, diese Funktion übernehmen könnten. In den folgenden Versuchen sollte deshalb geklärt werden, ob die antagonistische Wirkung auch auf einer Brühwurst-Schnittfläche erzielt werden kann. Die Brühwürste wurden nach einem im Stand derTechnik bekannten Verfahren hergestellt (Literaturstelle für die Herstellung von Brühwürsten: A. Fischer: Produktbezogene Technologie - Herstellung von Fleischerzeugnissen In: O. Prändl, A. Fischer, T. Schmidhofer, H.-J. Sinell (Hrsg.): Fleisch - Technologie und Hygiene der Gewinnung und Verarbeitung. Stuttgart: Ulmer, 1988, S. 505 ff).

Die Versuche wurden unter praxisorientierten Bedingungen durchgeführt. Um eine Schmierinfektion zu simulieren, wurden die Suspensionen mit den pathogenen Keimen nach dem Aufbringen auf die Schnittfläche mit einem Spatel verteilt. Die Kontamination der Brühwurst über die Aufschneidemaschine, wie sie in der Literatur angeführt wird (Schmidt, U. und Gardill, E., Jahresbericht der Bundesforschungsanstalt für Fleischforschung, S. C. 25 (1986), führte zu sehr ungleichmäßigen Verteilungen. Hierbei streifte das Messer beim Anschnitt vor allem am Randbereich bereits einen Großteil der Keime ab, teilweise konnten hierdurch bedingte relativ große Zonen (> 1 cm²) ermittelt werden, in denen der Testkeim nicht in Erscheinung trat.

Die Schutzkultur wurde anschließend mittels Druckluft über eine Sprühdüse aufgetragen. Das Aufsprühen der Keimsuspensionen führt zu einer gleichmäßigen Keimverteilung auf der Oberfläche. Nur eine gleichmäßige Verteilung der Schutzkultur kann eine optimale Hemmwirkung auf der gesamten Obertläche bewirken. Um zu verhindern, daß ein Teil der pathogenen Keime an die Verpackungsfolie anheftet und somit nicht erfaßt wird, wurde auf die jeweils kontaminierte Scheibe eine weitere Brühwurstscheibe aufgelegt. Danach wurden die Proben vakuumverpackt und bei den entsprechenden Temperaturen gelagert. Da für die mikrobiologischen Versuche die Oberfläche maßgebend war, wurden die Keimdichten in den nachfolgenden Versuchen flächenbezogen angegeben [KbE/cm²].

Figur 1 zeigt das Verhalten von *Lactococcus lactis subsp. lactis 1526* in vakuumverpacktem Brühwurstaufschnitt während einer dreiwöchigen Kühllagerung bei 6 °C und einer anschließend simulierten Kühlkettenunterbrechung (48h/ 22 °C).

### A) Hemmung des Salmonellen-Pools

Salmonellen setzen sich sehr schnell durch, wenn die erforderlichen Kühltemperaturen nicht eingehalten werden. Das Screening der Milchsäurebakterien-Isolate wurde deshalb vor allem auf die antagonistische Wirkung gegen die Salmonellen ausgerichtet. Die Abhängigkeit der Hemmung von der C-Quellen-Konzentration und der Einsaatdichte der Schutzkultur wurde in vakuumverpacktem Brühwurstaufschnitt untersucht, indem die Hemmwirkung von L. lactis subsp. lactis 1526 auf einen Salmonellen-Pool (bestehend aus 9 Wildstämmen, siehe Tabelle 1) bei unterschiedlichen Inokulationsdichten der Milchsäurebakterien-Kultur sowie bei verschiedenen Glucosekonzentrationen ermittelt wurde.

Die ungepökelten Brühwurstproben wurden mit insgesamt 4,4x10⁴ Salmonellen "schmierinfiziert", wobei die Überprüfung der Animpfdichte eine Wiederfindungsrate von 70 % ergab, was einer tatsächlichen Salmonellendichte von 700 KbE/cm² entspricht. Danach wurden *Lacctococcus lactis subsp. lactis 1526* in einer Dichte von 10⁶ KbE/cm² aufgesprüht. Über die Schutzkultursuspension wurde eine Glucosemenge von 0,7 mg/ cm² auf die Oberfläche aufgesprüht. Die angestrebte Laktokokkendichte von 10⁶ KbE/cm² wurde annähernd erreicht (1,1x10⁶ KbE/cm²). Die besprühten Scheiben wurden mit einer weiteren Scheibe überschichtet und vakuumverpackt. Anschließend wurden die Proben im Kühlbrutschrank bei 6 °C gelagert. Nach 14 Tagen wurde die Temperatur auf 22 °C erhöht, um eine KühlkettenUnterbrechung zu simulieren.

Während der Kühllagerung bei 6 °C veränderten sich die Keimdichten der Salmonellen und der Schutzkultur sowie der pH-Wert nur geringfügig (Figur 2). Nach der Temperaturerhöhung auf 22 °C kam es in den Proben ohne die Schutzkultur zu dem zu erwartenden Anstieg der Salmonellenkeimzahlen. Die Proben mit der Schutzkultur wurden dagegen deutlich gesäuert. Bereits nach 24 Stunden wurde ein Oberflächen-pH-Wert von 4,72 gemessen. Dadurch konnten die Salmonellen sehr deutlich gehemmt werden. Während der ersten 24 Stunden kam es nur zu einem geringen Anstieg der Zellzahlen von 170 auf 900 KbE/cm². Nach weiteren 24 Stunden konnten durchschnittlich noch 270 KbE/cm² nachgewiesen werden. Somit lag das Keimzahlniveau unter der Animpfdichte von 700 KbE/cm² (s. Figur 2).

Die bisher beschriebenen Versuche wurden bei einer Temperatur von 22 °C durchgeführt, um eine Kühlkettenunterbrechung zu simulieren. Ein mikrobielles Hygienerisiko kann jedoch auch dann auftreten, wenn die Kühltemperaturen von den geforderten 7 °C überschritten werden. Deshalb wurden weitere Untersuchungen bei einer Temperatur von 10 °C durchgeführt. Die angestrebte Animpfdichte der Salmonellen betrug bei diesen Versuchen 10³/cm²; die tatsächlich aufgefundene Dichte lag bei 1,36 x 10³ KbE/cm². Die Laktokokken wurden mit Keimdichten von 10⁵ KbE/cm² (Figur 3) angeimpft. Außerdem wurden 0,7mg/cm² Glukose aufgebracht.

In Figur 3 ist die Säuerungsaktivität der Schutzkultur sowie das Wachstumsverhalten der Salmonellen in den Proben mit sowie ohne Schutzkultur-Zugabe dargestellt. Die Salmonellenpopulation ohne die Schutzkultur konnte sich stark vermehren. In den Proben mit der Schutzkultur erreichte Lactococcus lactis subsp. lactis 1526 bereits am 7. Tag die maximale Keimzahl von 5,9x10⁷ KbE/cm² im weiteren Versuchsverlauf verringerten sich die Zellzahlen geringfügig. Der pH-Wert nahm in der ersten Woche deutlich ab, am 7.Tag wurden durchschnittliche Werte von 5,02 auf der Oberfläche gemessen, nach 14 Tagen wurden Werte von 4,76 erreicht. In Gegenwart der Schutzkultur konnten sich die Salmonellen während der 10 °C-Lagerung nur schwach vermehren. Am 14. Tag konnten lediglich Salmonellenkeimzahlen von 6,1 x 10³ KbE/cm² bestimmt werden. Nach der 14-tägigen Lagerung wurde die Temperatur auf 22 °C erhöht. Durch die Dominanz der Laktokokken konnten sich jedoch die Salmonellen nicht mehr durchsetzen.

### B) Hemmung von Staphylococcus aureus

Die Versuche zur Überprüfung der antagonistischen Wirkung von *L. lactis subsp. lactis 1526* gegenüber *Staphylococcus aureus* wurden methodisch entsprechend den unter A) beschriebenen Versuchen mit den Salmonellen durchgeführt. Die Ergebnisse des Versuches sind Figur 4 gezeigt. Aus Figur 4 wird deutlich, daß *L. lactis subsp. lactis 1526* in der Lage ist, bei einer Unterbrechung der Kühlung das Wachstum von *Staphylococcus aureus* stark zu reduzieren.

### C) Hemmung von Bacillus cereus

Die Versuche zur Überprüfung der antagonistischen Wirkung von *L. lactis subsp. lactis 1526* gegenüber *Bacillus cereus* wurde methodisch entsprechend den unter A) beschriebenen Versuchen mit den Salmonellen durchgeführt. Die Resultate sind in Figur 5 gezeigt. Aus den in Figur 5 gezeigten Ergebnissen wird deutlich, daß *L. lactis subsp. lactis 1526* in der Lage ist, bei einer Unterbrechung der Kühlung das Wachstum von *Bacillus cereus* vollständig zu unterbinden.

### D) Hemmung von Clostridium perfringens

Die Versuche zur Überprüfung der antagonistischen Wirkung von *L. lactis subsp. lactis 1526* gegenüber *Clostridium perfringens* wurden methodisch entsprechend den unter A) beschriebenen Versuchen mit den Salmonellen durchgeführt. Außerdem wurden 0,5 % Glucono-delta-Lacton (W/V) der Schutzkultur zugesetzt. Die Resultate sind aus Figur 6 ersichtlich. Es wird deutlich, daß *L. lactis subsp. lactis 1526* in der Gegenwart von Glucono-delta-Lacton in der Lage ist, eine Unterbrechung der Kühlung das Wachstum von *C. perfringens* zu unterbinden.

## Patentansprüche

1. Schutzkulturen zum Konservieren von Lebens- oder Futtermitteln, die unter Kühlung begrenzt haltbar sind, **dadurch gekennzeichnet, daß** sie nicht-pathogene Milchsäurebakterien mit den folgenden Eigenschaften umfassen:
a) Die Milchsäurebakterien zeigen bei Temperaturen unterhalb von 7 °C bis 8 °C keine sensorisch erfaßbare Stoffwechselaktivität;
b) Die Anzahl der Milchsäurebakterien, welche potentiell stoffwechselaktiv sind, nimmt bei Temperaturen unterhalb von 7 °C bis 8 °C über einen Zeitraum von einer bis zwei Wochen um weniger als zwei Zehnerpotenzen ab; und
c) Die Milchsäurebakterien hemmen bei Temperaturen von mindestens 7 °C bis 8 °C das Wachstum von toxinogenen und/oder toxiinfektiösen Bakterien.

2. Schutzkulturen nach Anspruch 1, **dadurch gekennzeichnet, daß** die Milchsäurebakterien zu den Gattungen *Pediococcus, Lactobacillus, Leuconostoc, Weissella, Enterococcus* und/oder *Lactococcus* gehören.

3. Schutzkulturen nach Anspruch 2, **dadurch gekennzeichnet, daß** die Milchsäurebakterien zu der Gattung *Lactococcus* gehören.

4. Schutzkulturen nach Anspruch 3, **dadurch gekennzeichnet, daß** die Milchsäurebakterien zu den Arten *Lactococcus lactis, Lactococcus garvieae, Lactococcus piscium, Lactococcus plantarum* und/oder *Lactococcus raffinolactis* gehören.

5. Schutzkulturen nach Anspruch 4, **dadurch gekennzeichnet, daß** die Milchsäurebakterien zur Art *Lactococcus lactis* gehören.

6. Schutzkulturen nach Anspruch 5, **dadurch gekennzeichnet, daß** die Milchsäurebakterien zu den Subspezies *Lactococcus lactis subsp*. *cremoris,* und/oder *Lactococcus lactis subsp. lactis* gehören.

7. Schutzkulturen nach Anspruch 6, **dadurch gekennzeichnet, daß** die Milchsäurebakterien zur Subspezies *Lactococcus lactis subsp. lactis* gehören.

8. Schutzkulturen nach Anspruch 7, **dadurch gekennzeichnet, daß** die Milchsäurebakterien zum Stamm *Lactococcus lactis subsp. lactis 1526* (DSM 12415) gehören.

9. Schutzkulturen nach den Ansprüchen 1 bis 8, **dadurch gekennzeichnet, daß** die Milchsäurebakterien bei Temperaturen von mindestens 7 °C bis 8 °C oder darüber das Wachstums von toxinogenen und/oder toxiinfektiösen Bakterien durch die Produktion organischer Säuren hemmen.

10. Schutzkulturen nach den Ansprüchen 1 bis 9, **dadurch gekennzeichnet, daß** die Milchsäurebakterien bei Temperaturen von mindestens 7 °C bis 8 °C oder darüber eine sensorisch erfaßbare Stoffwechselaktivität zeigen.

11. Schutzkulturen nach den Ansprüchen 1 bis 10, **dadurch gekennzeichnet, daß** die Temperaturen 7 °C betragen.

12. Milchsäurebakterien, **dadurch gekennzeichnet, daß** die Milchsäurebakterien zum Stamm *Lactococcus lactis subsp. lactis 1526* (DSM 12415) gehören.

13. Verwendung der Schutzkulturen nach den Ansprüchen 1 bis 11 zur Konservierung von Lebens- oder Futtermitteln, die unter Kühlung begrenzt haltbar sind.

14. Lebens- oder Futtermittel, **dadurch gekennzeichnet, daß** es die Schutzkulturen nach den Ansprüchen 1 bis 11 enthält.

15. Lebensmittel nach Anspruch 14, **dadurch gekennzeichnet, daß** das Lebensmittel aus der Gruppe bestehend aus Fleisch oder Fleischerzeugnissen, Fisch oder Fischerzeugnissen, Feinkostsalaten und vorgegarten Fertiggerichten ausgewählt ist.
